# EUROPEAN PATENT APPLICATION

(11) **EP 2 258 686 A1**
(43) Date of publication of application: **08.12.2010**
(21) Application number: 09160246.6
(22) Date of filing: 14.05.2009
(51) Int. Cl.: C07D 213/77, C09B 35/031, C09D 11/00

(54) **Monoazo compounds**

(71) Applicant: CLARIANT INTERNATIONAL LTD, 4132 Muttenz 1 (CH)
(72) Inventor: Nusser, Rainer, 79395 Neuenburg (DE); Geiger, Ulrich, 79395 Neuenburg/Rhein (DE); Hasemann, Ludwig, 79379 Muellheim-Niederweiler (DE)
(74) Representative: Jacobi, Carola

(57) **Abstract**

Acid dyes, a process for their preparation and their use for dyeing organic substrates of the general formula (I) 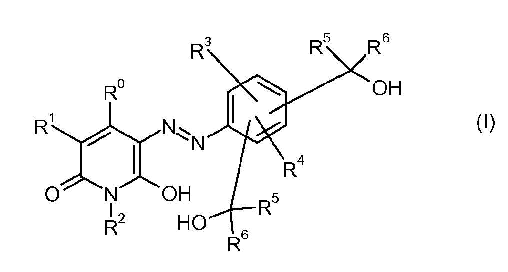 wherein the substituents have the meaning as indicated in the claims.

## Description

The invention relates to novel acid dyes, a process for their preparation and their use for dyeing organic substrates.

Acid dyes are known. However, there is still a need for acid dyes with improved properties.

The invention provides compounds of the general formula (I) 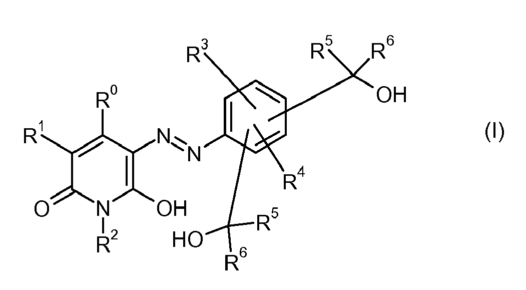
- R⁰: signifies a substituted C₁ to C₄ alkyl group or an unsubstituted C₁ to C₄ alkyl group,
- R¹: signifies H, a substituted C₁ to C₄ alkyl group or an unsubstituted C₁ to C₄ alkyl group, a sulpho group, -CO-NH₂, -CO-NH-(C₁ to C₄ alkyl) or CN,
- R²: signifies H, a substituted C₁ to C₄ alkyl group or an unsubstituted C₁ to C₄ alkyl group,
- R³: signifies H, a sulpho group, a substituted C₁ to C₄ alkyl group or an unsubstituted C₁ to C₄ alkyl group, a substituted C₁ to C₄ alkoxy group or an unsubstituted C₁ to C₄ alkoxy group,
- R⁴: signifies H, a substituted C₁ to C₄ alkyl group or an unsubstituted C₁ to C₄ alkyl group, a substituted C₁ to C₄ alkoxy group or an unsubstituted C₁ to C₄ alkoxy group,
- R⁵: signifies H,
- R⁶: signifies a substituted C₁ to C₉ alkyl group or an unsubstituted C₁ to C₉ alkyl group, an unsubstituted aryl group or a substituted aryl group,

By preference, the sum of carbon atoms of R⁵ and R⁶ together is at least 4 carbon atoms, more preferred R⁵ and R⁶ have together at least 5 carbon atoms. Even more preferred, the sum of carbon atoms of R⁵ and R⁶ together is 5 or 6 or 7 or 8 or 9 carbon atoms.

The preferred compounds of formula (I) bear at least one anionic substituent, preferably 1 or 2 or 3 anionic substituents, of which 2 anionic substituents are very particularly preferred.

The at least one anionic substituent in the compounds of formula (I) are located by preference in one of the substituents R¹ and/or R³, more preferred, the at least one anionic substituent is located in one of the substituents R². Located by preference in one of the substituents may also mean that this substituent is the anionic group.

Preferred anionic substituents are carboxyl and/or sulpho groups, and sulpho groups are particularly preferred.

The preferred substituents of the substituted C₁ to C₄ alkyl groups are selected from the following substituents -OH, -O(C₁ to C₄ - Alkyl), -SO₃H, -COOH, -NH(C₁ to C₄ - Alkyl). The more preferred substituents of the substituted C₁ to C₄ alkyl groups are selected from the following substituents -OH, -O(C₁ to C₄ - Alkyl), -SO₃H, -COOH, -NH(C₁ to C₄ - Alkyl). The alkyl groups groups are branched or linear. The most preferred alkyl groups are methyl, ethyl, propyl, iso-proply, butyl, iso-butyl (2-Methylpropyl), pentyl, iso-pentyl (3-Methylbutyl, hexyl, heptyl, octyl, or nonyl

The preferred substituents of the substituted C₁ to C₄ alkoxy group are selected from the following substituents -OH, -O(C₁ to C₄ - Alkyl), -SO₃H, -COOH, -NH(C₁ to C₄ - Alkyl). The alkoxy groups are branched or linear.

Preferred substituents of the substituted aryl groups are selected from the following substituents -OH, -O(C₁ to C₄ - Alkyl), -SO₃H, substituted C₁ to C₄ alkyl groups, unsubstituted alkyl groups, a substituted C₁ to C₄ alkoxy group and an unsubstituted C₁ to C₄ alkoxy group.

In preferred compounds of the general formula (I)
- R⁰: signifies an unsubstituted C₁ to C₂ alkyl group,
- R¹: signifies a substituted C₁ to C₂ alkyl group or an unsubstituted C₁ to C₂ alkyl group, a sulpho group, -CO-NH₂, -CO-NH-(C₁ to C₂ alkyl) or CN,
- R²: signifies a substituted C₁ to C₃ alkyl group or an unsubstituted C₁ to C₃ alkyl group,
- R³: signifies H, a sulpho group, an unsubstituted C₁ to C₂ alkyl group, a an unsubstituted C₁ to C₂ alkoxy group,
- R⁴: signifies H, an unsubstituted C₁ to C₂ alkyl group, a an unsubstituted C₁ to C₂ alkoxy group,
- B: a group with the formula -SO₂-, -CR⁵R⁶-, wherein
- R⁵: signifies H, substituted C₁ to C₉ alkyl group or an unsubstituted C₁ to C₉ alkyl group,
- R⁶: signifies a substituted C₁ to C₉ alkyl group or an unsubstituted C₁ to C₉ alkyl group, an unsubstituted aryl group or a substituted aryl group or R⁵ and R⁶ form together a five or six membered cyclo aliphatic ring, wherein the five or six membered rings are substituted by a C₁ to C₄ alkyl group or the five or six membered rings are not further substituted.

In even more preferred compounds of the general formula (I)
- R⁰: signifies a methyl group,
- R¹: signifies -CH₂-SO₃H, -CONH₂ or -CN,
- R²: signifies a ethyl group, -CH₂-CH₂-CH₂-NH-CH₃, -CH₂-CH₂-COOH, or - CH₂-CH₂-CH₂-O-CH₂-CH₂-CH₂-CH₃
- R³: signifies H, methyl, methoxy or a sulpho group
- R⁴: signifies H, methyl or a methoxy group
- B: a group with the formula -SO₂- or -CR⁵R⁶-, wherein
- R⁵: signifies H, methyl or a ethyl group,
- R⁶: signifies an unsubstituted C₁ to C₄ alkyl group, a unsubstituted aryl group or a substituted aryl group or R⁵ and R⁶ form together a six membered cyclo aliphatic ring, wherein the six membered rings are not further substituted.

In the most preferred compounds of the general formula (I)
- R⁰: signifies a methyl group,
- R¹: signifies -CH₂-SO₃H, -CONH₂ or -CN preferably a -CH₂-SO₃H group,
- R²: signifies a ethyl group, -CH₂-CH₂-CH₂-NH-CH₃, -CH₂-CH₂-COOH, or -CH₂-CH₂-CH₂-O-CH₂-CH₂-CH₂-CH₃, preferably a ethyl group,
- R³: signifies H, methyl, methoxy or a sulpho group, preferably H, methyl,
- R⁴: signifies H, methyl or a methoxy group preferably H,
- B: a group with the formula -SO₂- or -CR⁵R⁶-, wherein
- R⁵: signifies H,
- R⁶: signifies a unsubstituted aryl group or a substituted aryl group, preferably a unsubstituted aryl group wherein the aryl group is phenyl group.

The invention also provides a process for preparing compounds of the formula (I). The present invention's compounds of the formula (I) can be prepared under conventional conditions in conventional processes.

This process for the preparation of compounds of formula (I) comprises reacting the diazonium salt of a amine of formula (II), 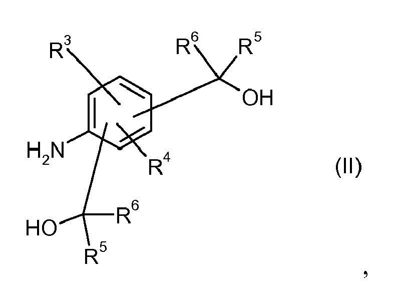 with one equivalent compound of formula (III). 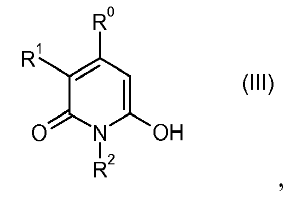 in which R⁰, R¹, R², R³, R⁴, R⁵ and R⁶ are defined as above defined.

Diazotisation and coupling may be effected in accordance with conventional methods. The coupling reaction advantageously is carried out in an aqueous reaction medium in a temperature range of from 0 - 60°C, preferably at 0- 40°C, more preferred at 0 - 10°C, even more preferred at 0 - 5°C and in a pH range of from 2 to 9, preferably at pH 3 to 6. All temperatures are given in degrees Celsius.

The reaction mixtures comprising compounds of formula (I) thus obtained may be converted into stable liquid formulations with improved long term stability by desalting by ultra filtration.

The compounds of formula (I) thus obtained may be isolated in accordance with known methods.

The starting compounds, the amines of formula (II) and of compounds of formula (III), are either known or may be prepared in accordance with known methods from available starting materials. Suitable methods are described e.g. in DE399149; DE505475; DE1220863; DE1793020 (GB1129306), DE3226889, DE4014847.

However, novel amines according to the formula (II) may be prepared according the methods disclosed in DE399149; DE505475; DE1220863; DE1793020 (GB1129306), DE3226889, DE4014847, thus starting form aldehydes (when R⁵ is H and R⁶ is different from H) of the formula 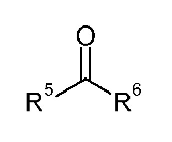 by reacting at elevated temperature and elevated pressure under acidic condition with one equivalents of an aromatic amine of the formula 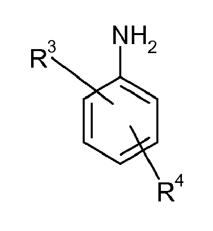 forming the amine of the formula (II)

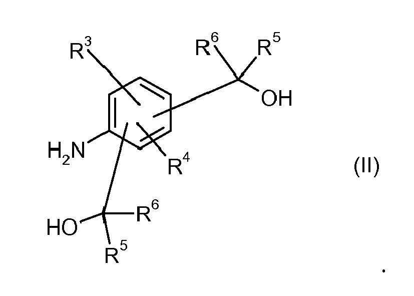

The reaction mixture is heated in a closed autoclave at 120°C-250°C, preferably 140°C-200°C, more preferably 140°C to 150°C the reaction mixture is kept at this temperature for 3-8 hours, preferably for 4-5 hours. The elevated temperature leads in this closed autoclave to the elevated pressure. Alternatively the synthesis may be performed in the melt of the aminocompound-hydrochloride adding the aldehyde at elevated temperature 200 to 250°C and the pressure is atmospheric pressure.

The dyes of the formula (I) can be isolated from the reaction medium by conventional processes, for example by salting out with an alkali metal salt, filtering and drying, if appropriate under reduced pressure and at elevated temperature.

Depending on the reaction and/or isolation conditions, the dyes of the formula (I) can be obtained as free acid, as salt or as mixed salt which contains for example one or more cations selected from alkali metal ions, for example the sodium ion, or an ammonium ion or alkylammonium cation, for example mono-, di- or trimethyl- or -ethylammonium cations. The dye can be converted by conventional techniques from the free acid into a salt or into a mixed salt or vice versa or from one salt form into another. If desired, the dyes can be further purified by diafiltration, in which case unwanted salts and synthesis by-products are separated from the crude anionic dye.

The removal of unwanted salts and synthesis by-products and partial removal of water from the crude dye solution is carried out by means of a semipermeable membrane by applying a pressure whereby the dye is obtained without the unwanted salts and synthesis by-products as a solution and if necessary as a solid body in a conventional manner.

The dyes of the formula (I) and their salts are particularly suitable for dyeing or printing fibrous material consisting of natural or synthetic polyamides in yellow to greenish yellow shades. The dyes of the formula (I) and their salts are suitable for producing Inkjet printing inks and for using these Inkjet printing inks to print fibrous material which consists of natural or synthetic polyamides or cellulose (paper for example).

The invention accordingly provides from another aspect for the use of the dyes of the formula (I), their salts and mixtures for dyeing and/or printing fibrous materials consisting of natural or synthetic polyamides. A further aspect is the production of Inkjet printing inks and their use for printing fibrous materials consisting of natural or synthetic polyamides.

Dyeing is carried out as per known processes, see for example the dyeing processes described in Ullmanns Encyklopädie der technischen Chemie, 4th Edition, 1982, Volume 22, pages 658-673 or in the book by M. Peter and H.K. Rouette, Grundlagen der Textilveredlung, 13th Edition, 1989, pages 535-556 and 566-574. Preference is given to dyeing in the exhaust process at a temperature of 30 to 140°C, more preferably 80 to 120°C and most preferably at a temperature of 80 to 100°C, and at a liquor ratio in the range from 3:1 1 to 40:1.

The substrate to be dyed can be present in the form of yarn, woven fabric, loop-formingly knitted fabric or carpet for example. Fully fashioned dyeings are even permanently possible on delicate substrates, examples being lambswool, cashmere, alpaca and mohair. The dyes of the invention are particularly useful for dyeing fine-denier fibres (microfibres).

The dyes according to the present invention and their salts are highly compatible with known acid dyes. Accordingly, the dyes of the formula (I), their salts or mixtures can be used alone in a dyeing or printing process or else as a component in a combination shade dyeing or printing composition together with other acid dyes of the same class, i.e. with acid dyes possessing comparable dyeing properties, such as for example fastness properties and exhaustion rates from the dyebath onto the substrate. The dyes of the present invention can be used in particular together with certain other dyes having suitable chromophores. The ratio in which the dyes are present in a combination shade dyeing or printing composition is dictated by the hue to be obtained.

The novel dyes of the formula (I), as stated above, are very useful for dyeing natural and synthetic polyamides, i.e. wool, silk and all nylon types, on each of which dyeings having a high fastness level, especially good light fastness and good wet fastnesses (washing, alkaline perspiration) are obtained. The dyes of the formula (I) and their salts have a high rate of exhaustion. The ability of the dyes of the formula (I) and their salt to build up is likewise very good. On-tone dyeings on the identified substrates are of outstanding quality. All dyeings moreover have a constant hue under artificial light. Furthermore, the fastness to decating and boiling is good.

One decisive advantage of the novel dyes is that they are metal free and provide very level dyeings.

The compounds according to the invention can be used as an individual dye or else, owing to their good compatibility, as a combination element with other dyes of the same class having comparable dyeing properties, for example with regard to general fastnesses, exhaustion value, etc. The combination shade dyeings obtained have similar fastnesses to dyeings with the individual dye.

The invention's dyes of the formula (I) can also be used as yellow components in trichromatic dyeing or printing. Trichromatic dyeing or printing can utilize all customary and known dyeing and printing processes, such as for example the continuous process, exhaustion process, foam dyeing process and Ink-Jet process.

The composition of the individual dye components in the trichromatic dye mixture used in the process of the invention depends on the desired hue. A brown hue for example preferably utilizes 20 - 40% by weight of a yellow component, 40 - 60% by weight of the invention's orange or red component and 10 - 20% by weight of a blue component.

The yellow component, as described above, can consist of a single component or of a mixture of different orange individual components conforming to the formula (I). Preference is given to double and triple combinations.

Particularly preferred red and/or blue components are described in W02002/46318 or WO99/51681 respectively.

The following examples further serve to illustrate the invention. In the Examples all parts and all percentages are by weight or volume, and the temperatures given are in degrees Celsius, unless indicated to the contrary.

The invention will now be illustrated by the following Examples in which all parts are by weight and all temperatures are in degrees Celsius.

### Example 1: (method A)

212 g benzaldehyde (2 mol), 107 g o-xylidine (1 mol), 115 g hydrochloric acid (ca. 30%) and 300 ml water were heated in an autoclave at 140°C for 6 hours.

The reaction mixture was poured on 1 kg ice and 135 g sodium hydroxide solution (30%). The organic layer was separated and the excess of o-xylidine separated with toluene. The residue was re-crystallized from toluene and the press cake washed with cold alcohol. A compound of the formula (1) was obtained:

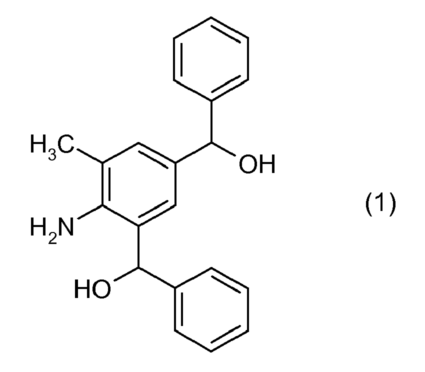

### 1-hydroxy-(3-methyl-4-amino-5-(1-hydroxy-phenylmethane)-phenyl)-phenylmethane

### Example 2: (method B)

130 g (1mol) of aniline hydrochloride are melted in a 0.5-1 reaction vessel under nitrogen at 220°C and 256 g (2 mol) of 2-Ethylhexanal is slowly added thereto while stirring over a period of 4 hour.

The temperature of the melt falls from initially ca. 200 °C to 185°C because of the reflux. The temperature is kept for one hour at 185°C and the hot melt is poured on a mixture of 1.6 kg ice and 1.05 kg of sodium hydroxide solution (30%).

The organic layer is separated and washed free from salt with demineralised water.

The residue was re-crystallized from toluene and the press cake is washed with cold ethanol. A compound of the formula (2) was obtained: 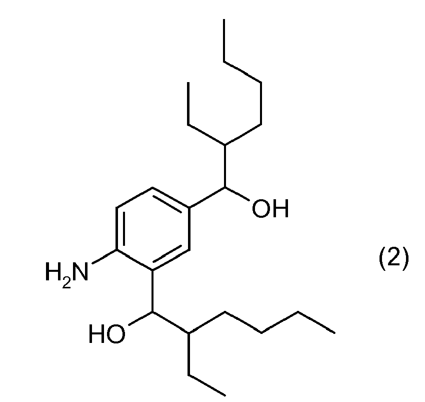 1-hydroxy-1-(3-(1-hydroxy-2-ethyl-hexane)-4-aminophenyl)-2-ethyl-hexane

**Table 1: Synthesis of the amines starting with aldehydes**

| | | | |
|---|---|---|---|
| 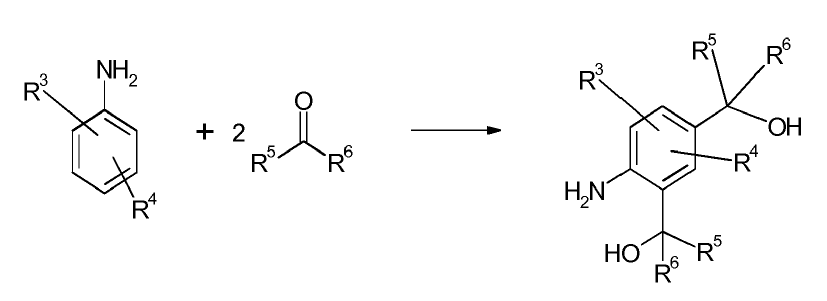 | | | |

| **Nr.** | 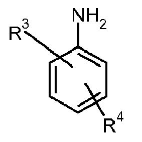 | **R⁵** | **R⁶** |
|---|---|---|---|
| 3 | 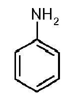 | H | CH₂CH₂CH₂CH₃ |
| 4 | 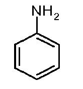 | H | CH₂CH(CH₃)₂ |
| 5 | 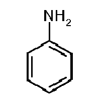 | H | CH(CH₃)CH₂CH₃ |
| 6 | 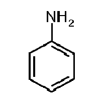 | H | CH(CH₃)CH₂CH₂CH₃ |
| 7 | 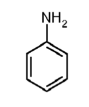 | H | (CH₂)₅CH₃ |
| 8 | 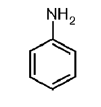 | H | (CH₂)₄CH₃ |
| 9 | 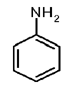 | H | CH(CH₂CH₃)(CH₂)₃CH₃ |
| 10 | 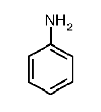 | H | (CH₂)₆CH₃ |
| 11 | 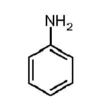 | H | (CH₂)₇CH₃ |
| 12 | 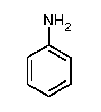 | H | (CH₂)₈CH₃ |
| 13 | 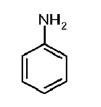 | H | CH₂-Ph |
| 14 | 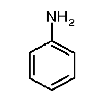 | H | Ph |
| 15 | 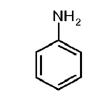 | H | 4-Ph-CH₃ |
| 16 | 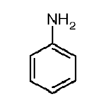 | H | 4- Ph-CH(CH₃)₂ |
| 17 | 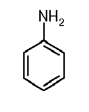 | H | 4- Ph-t-Bu |
| 18 | 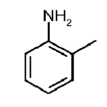 | H | CH₂CH(CH₃)₂ |
| 19 | 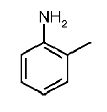 | H | CH(CH₂CH₃)₂ |
| 20 | 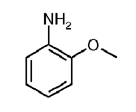 | H | Phenyl |
| 21 | 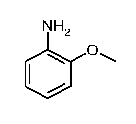 | H | CH(CH₂CH₃)₂ |
| 22 | 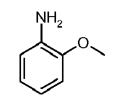 | H | CH(CH₂CH₃)(CH₂)₃CH₃ |

### Example 23:

35 Parts (0.1 mol) of 1-hydroxy-1-(3-(1-hydroxy-2-ethyl-hexane)-4-aminophenyl)-2-ethylhexane (amine-example 2) are diazotised according to known methods with 6.9 parts (0.1 mol) of sodium nitrite at 0-5°C in 200 parts of water and 30 parts of hydrochloric acid (ca. 30%).
24.7 Parts (0.1 mol) of a compound of the formula 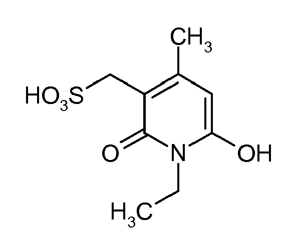 dissolved in 250 parts of water are added over 30 minutes to the ice cold tetrazotised solution. By the addition of 30% NaOH solution the pH is brought to 3-4.5 yielding a dyestuff of formula (5) and the dyestuff is in solution. λ max = 451 nm.

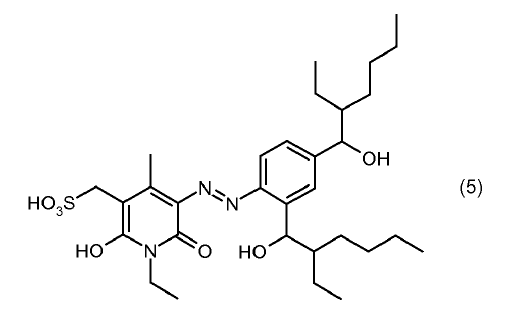

The dyestuff can be isolated by concentration under vacuum or by precipitation in aceton/alcohol.

The reaction mixture however can be used directly for dyeing without isolation the product. The dyestuff of formula (5) has surprisingly high solubility in water and gives yellow dyeings with very good fastness properties.

### Example 24:

31.9 Parts (0.1 mol) of 1-hydroxy-(3-methyl-4-amino-5-(1-hydroxy-phenylmethane)-phenyl)-phenylmethane (amine-example 1) are diazotised according to known methods with 6.9 parts (0.1 mol) of sodium nitrite at 0-5°C in 200 parts of water and 30 parts of hydrochloric acid (ca. 30%).

24.7 Parts (0.1 mol) of a compound of the formula 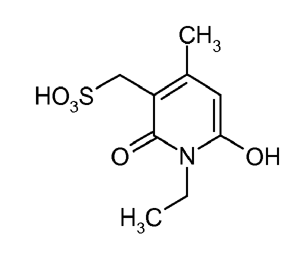 dissolved in 250 parts of water are added over 30 minutes to the ice cold tetrazotised solution. By the addition of 30% NaOH solution the pH is brought to 3-4.5 yielding a dyestuff of formula (6) and the dyestuff is in solution. λ max = 453 nm.

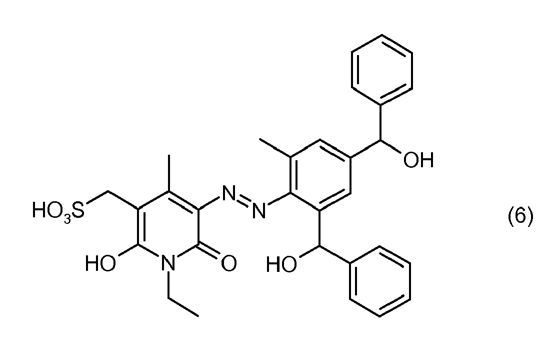

The dyestuff can be isolated by concentration under vacuum or by precipitation in aceton/alcohol.

The reaction mixture however can be used directly for dyeing without isolation the product. The dyestuff of formula (6) has high solubility in water and gives yellow dyeings with surprisingly very good fastness properties.

### Table 2: synthesis of the dyesstuff with the amines from Table 1

The following compounds shown in the table 2 were synthesized according to the example 23 or 24 using the amine 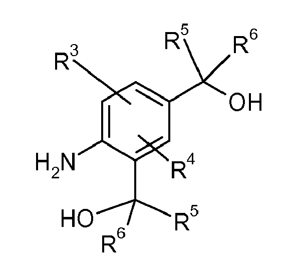 as diazo component and reacted with coupling component 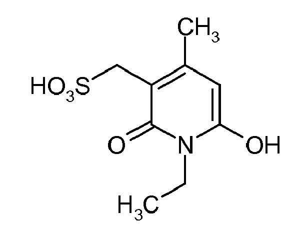 wherein a compound of the following formula was obtained: 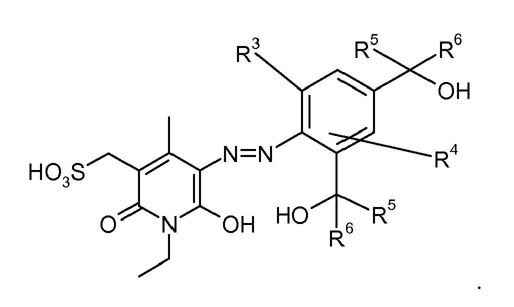 λ max (lambda max) is indicated in nm (nano meters; measured in 1% acetic acid solution).

| Dye-stuff-nr. | Amine | Compound-nr. | λ max |
|---|---|---|---|
| 25 | 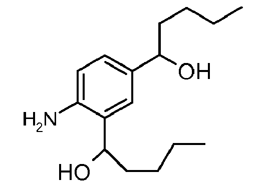 | 3 | 433 |
| 26 | 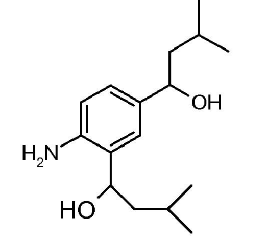 | 4 | 434 |
| 27 | 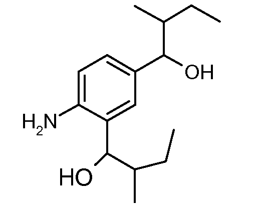 | 5 | 435 |
| 28 | 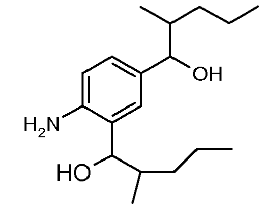 | 6 | 435 |
| 29 | 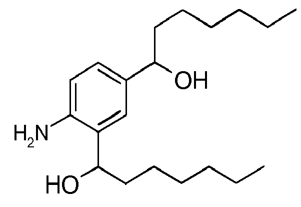 | 7 | 436 |
| 30 | 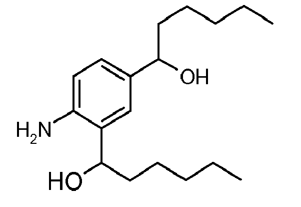 | 8 | 437 |
| 31 | 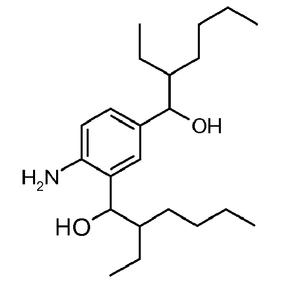 | 9 | 436 |
| 32 | 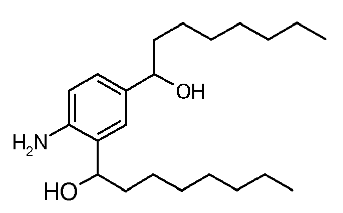 | 10 | 436 |
| 33 | 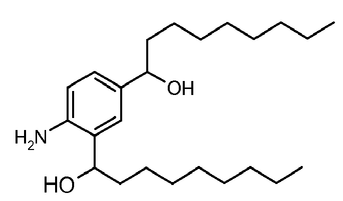 | 11 | 435 |
| 34 | 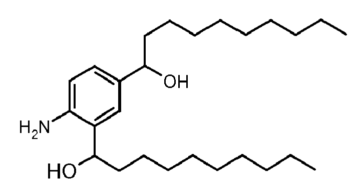 | 12 | 437 |
| 35 | 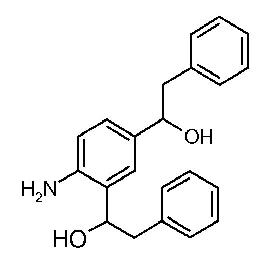 | 13 | 436 |
| 36 | 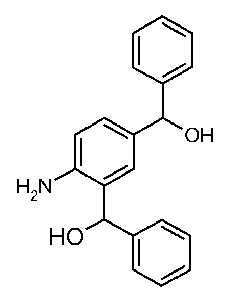 | 14 | 436 |
| 37 | 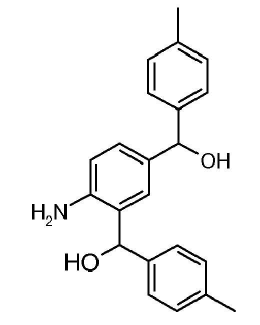 | 15 | 435 |
| 38 | 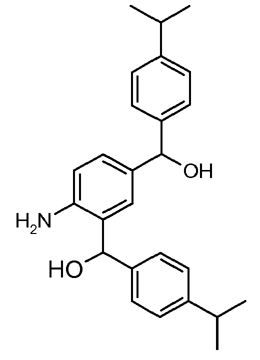 | 16 | 434 |
| 39 | 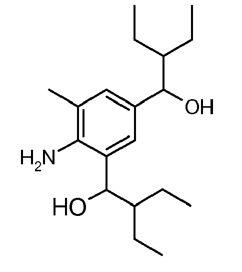 | 19 | 440 |
| 40 | 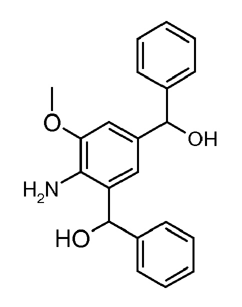 | 20 | 443 |
| 41 | 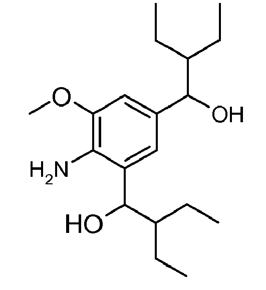 | 21 | 467 |
| 42 | 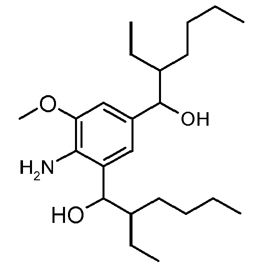 | 22 | 466 |

### USE EXAMPLE A

A dyebath at 40°C, consisting of 2000 parts of water, 1 part of a weakly cation-active levelling agent which is based on an ethoxylated aminopropyl fatty acid amide and which has affinity for dye, 0.25 part of the dye of Preparation Example 23 and adjusted to pH 5 with 1-2 parts of 40% acetic acid is entered with 100 parts of nylon-6 fabric. After 10 minutes at 40°C, the dyebath is heated to 98°C at a rate of 1°C per minute and then left at the boil for 45-60 minutes. Thereafter it is cooled down to 70°C over 15 minutes. The dyeing is removed from the bath, rinsed with hot and then with cold water and dried. The result obtained is a yellow polyamide dyeing possessing good light and wet fastnesses.

### USE EXAMPLE B

A dyebath at 40°C, consisting of 2000 parts of water, 1 part of a weakly cation-active levelling agent which is based on an ethoxylated aminopropyl fatty acid amide and which has affinity for dye, 0.3 part of the dye of Preparation Example 23 and adjusted to pH 5.5 with 1-2 parts of 40% acetic acid is entered with 100 parts of nylon-6,6 fabric. After 10 minutes at 40°C, the dyebath is heated to 120°C at a rate of 1.5°C per minute and then left at this temperature for 15-25 minutes. Thereafter it is cooled down to 70°C over 25 minutes. The dyeing is removed from the dyebath, rinsed with hot and then with cold water and dried. The result obtained is a yellow polyamide dyeing with good levelness and having good light and wet fastnesses.

### USE EXAMPLE C

A dyebath at 40°C, consisting of 4000 parts of water, 1 part of a weakly amphoteric levelling agent which is based on a sulphated, ethoxylated fatty acid amide and which has affinity for dye, 0.4 part of the dye of Preparation Example 23 and adjusted to pH 5 with 1-2 parts of 40% acetic acid is entered with 100 parts of wool fabric. After 10 minutes at 40°C, the dyebath is heated to boiling at a rate of 1°C per minute and then left at the boil for 40-60 minutes. Thereafter it is cooled down to 70°C over 20 minutes. The dyeing is removed from the bath, rinsed with hot and then with cold water and dried. The result obtained is a yellow wool dyeing possessing good light and wet fastnesses.

### USE EXAMPLE D

| | | |
|---|---|---|
| 100 | parts | of a woven nylon-6 material are padded with a 50°C liquor consisting of |
| 40 | parts | of the dye of Preparation Example 23, |
| 100 | parts | of urea, |
| 20 | parts | of a nonionic solubilizer based on butyldiglycol, |
| 15-20 | parts | of acetic acid (to adjust the pH to 4), |
| 10 | parts | of a weakly cation-active levelling agent which is based on an ethoxylated aminopropyl fatty acid amide and has affinity for dye, and |
| 810-815 | parts | of water (to make up to 1000 parts of padding liquor). |

The material thus impregnated is rolled up and left to dwell in a steaming chamber under saturated steam conditions at 85-98°C for 3-6 hours for fixation. The dyeing is then rinsed with hot and cold water and dried. The result obtained is a yellow nylon dyeing having good levelness in the piece and good light and wet fastnesses.

### USE EXAMPLE E

A textile cut pile sheet material composed of nylon-6 and having a synthetic base fabric is padded with a liquor containing per 1000 parts

| | |
|---|---|
| 1 | part of dye of Preparation Example 23 |
| 4 | parts of a commercially available thickener based on carob flour ether |
| 2 | parts of a nonionic ethylene oxide adduct of a higher alkylphenol |
| 1 | part of 60% acetic acid. |

This is followed by printing with a paste which per 1000 parts contains the following components:

| | |
|---|---|
| 20 | parts of commercially available alkoxylated fatty alkylamine (displace product) |
| 20 | parts of a commercially available thickener based on carob flour ether. |

The print is fixed for 6 minutes in saturated steam at 100°C, rinsed and dried. The result obtained is a level-coloured cover material having a yellow and white pattern.

### USE EXAMPLE F

100 parts of a chrome-tanned and synthetically retanned shave-moist grain leather are dyed for 30 minutes in a bath of 300 parts of water and 2 parts of the dye of Preparation Example 23 at 55°C. After addition of 4 parts of a 60% emulsion of a sulphited fish oil, the leather is fatliquored for 45 minutes. It is then acidified with 8.5% formic acid and milled for 10 minutes (final pH in the bath 3.5-4.0). The leather is then rinsed, allowed to drip dry and finished as usual. The result obtained is a leather dyed in a level clear orange hue with good fastnesses.

Use Examples A to F can also be carried out with dyes 24 to 42 with similar results.

### USE EXAMPLE G

3 parts of the dye of Preparation Example 23 are dissolved in 82 parts of demineralized water and 15 parts of diethylene glycol at 60°C. Cooling down to room temperature gives an orange printing ink which is very highly suitable for ink jet printing on paper or polyamide and wool textiles.

Use Example G can also be carried out with dyes 24 to 42 with similar results.

### USE EXAMPLE H

A dyebath consisting of 1000 parts of water, 80 parts of calcined Glauber salt, 1 part of sodium nitrobenzene-3-sulphonate and 1 part of dye from Example 23 is heated to 80°C in the course of 10 minutes. Then, 100 parts of mercerized cotton are added. This is followed by dyeing at 80°C for 5 minutes and then heating to 95°C in the course of 15 minutes. After 10 minutes at 95°C, 3 parts of sodium carbonate are added, followed by a further 7 parts of sodium carbonate after 20 minutes and another 10 parts of sodium carbonate after 30 minutes at 95°C. Dyeing is subsequently continued at 95°C for 60 minutes. The dyed material is then removed from the dyebath and rinsed in running demineralized water for 3 minutes. This is followed by two washes for 10 minutes in 5000 parts of boiling demineralized water at a time and subsequent rinsing in running demineralized water at 60°C for 3 minutes and with cold tap water for one minute. Drying leaves a brilliant yellow cotton dyeing having good fastnesses.

### USE EXAMPLE I

0.2 part of the dye of Preparation Example 23 is dissolved in 100 parts of hot water and the solution is cooled down to room temperature. This solution is added to 100 parts of chemically bleached sulphite pulp beaten in 2000 parts of water in a Hollander. After 15 minutes of commixing the stuff is sized with resin size and aluminium sulphate in a conventional manner. Paper produced from this stuff has a yellow shade with good wet fastnesses.

Use Examples H and I can also be carried out with dyes 24 to 42 with similar results.

## Claims

1. Compounds of the general formula (I) 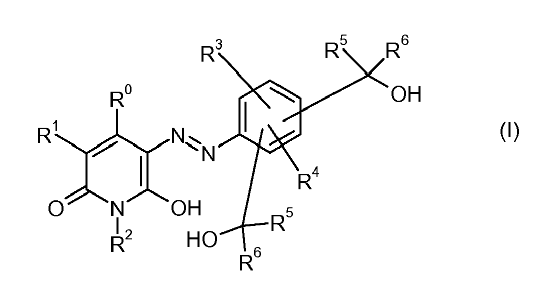 wherein
R⁰ signifies a substituted C₁ to C₄ alkyl group or an unsubstituted C₁ to C₄ alkyl group,
R¹ signifies H, a substituted C₁ to C₄ alkyl group or an unsubstituted C₁ to C₄ alkyl group, a sulpho group, -CO-NH₂, -CO-NH-(C₁ to C₄ alkyl) or CN,
R² signifies H, a substituted C₁ to C₄ alkyl group or an unsubstituted C₁ to C₄ alkyl group,
R³ signifies H, a sulpho group, a substituted C₁ to C₄ alkyl group or an unsubstituted C₁ to C₄ alkyl group, a substituted C₁ to C₄ alkoxy group or an unsubstituted C₁ to C₄ alkoxy group,
R⁴ signifies H, a substituted C₁ to C₄ alkyl group or an unsubstituted C₁ to C₄ alkyl group, a substituted C₁ to C₄ alkoxy group or an unsubstituted C₁ to C₄ alkoxy group,
B a group with the formula -SO₂-, -NH-CO-NH-, -CR⁵R⁶-, wherein
R⁵ signifies H, substituted C₁ to C₉ alkyl group or an unsubstituted C₁ to C₉ alkyl group,
R⁶ signifies H, a substituted C₁ to C₉ alkyl group or an unsubstituted C₁ to C₉ alkyl group, an unsubstituted aryl group or a substituted aryl group or R⁵ and R⁶ form together a five or six membered cyclo aliphatic ring, wherein the five or six membered rings are substituted by a C₁ to C₄ alkyl group or the five or six membered rings are not further substituted.

2. Compounds according to claim 1 **characterized in that** the compounds of formula (I) bear at least one anionic substituent

3. Compounds according to claim 2 **characterized in that**
R⁰ signifies an unsubstituted C₁ to C₂ alkyl group,
R¹ signifies a substituted C₁ to C₂ alkyl group or an unsubstituted C₁ to C₂ alkyl group, a sulpho group, -CO-NH₂, -CO-NH-(C₁ to C₂ alkyl) or CN,
R² signifies a substituted C₁ to C₃ alkyl group or an unsubstituted C₁ to C₃ alkyl group,
R³ signifies H, a sulpho group, an unsubstituted C₁ to C₂ alkyl group, a an unsubstituted C₁ to C₂ alkoxy group,
R⁴ signifies H, an unsubstituted C₁ to C₂ alkyl group, a an unsubstituted C₁ to C₂ alkoxy group,
B a group with the formula -SO₂-, -CR⁵R⁶-, wherein
R⁵ signifies H, substituted C₁ to C₉ alkyl group or an unsubstituted C₁ to C₉ alkyl group,
R⁶ signifies a substituted C₁ to C₉ alkyl group or an unsubstituted C₁ to C₉ alkyl group, an unsubstituted aryl group or a substituted aryl group or R⁵ and R⁶ form together a five or six membered cyclo aliphatic ring, wherein the five or six membered rings are substituted by a C₁ to C₄ alkyl group or the five or six membered rings are not further substituted.

4. Compounds according to claim 3 **characterized in that**
R⁰ signifies a methyl group,
R¹ signifies -CH₂-SO₃H, -CONH₂ or -CN,
R² signifies a ethyl group, -CH₂-CH₂-CH₂-NH-CH₃, -CH₂-CH₂-COOH, or - CH₂-CH₂-CH₂-O-CH₂-CH₂-CH₂-CH₃
R³ signifies H, methyl, methoxy or a sulpho group
R⁴ signifies H, methyl or a methoxy group
B a group with the formula -SO₂- or -CR⁵R⁶-, wherein
R⁵ signifies H, methyl or a ethyl group,
R⁶ signifies an unsubstituted C₁ to C₄ alkyl group, a substituted aryl group or R⁵ and R⁶ form together a six membered cyclo aliphatic ring, wherein the six membered rings are not further substituted.

5. Process for preparing compounds of the formula (I) according to Claim 1, **characterized in that** the amine function of the compound of the formula (II) 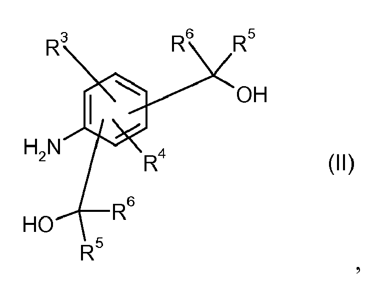 is diazotized and coupled onto one equivalent of a compound of the formula (III) 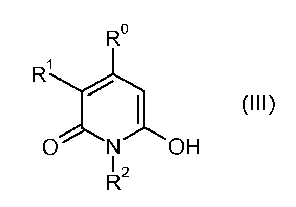 where the substituents R⁰, R¹, R², R³, R⁴, R⁵ and R⁶ are each as defined above.

6. Use of the compounds of the formula (I) according to Claim 1 for dyeing and/or printing organic substrates.

7. Use of compounds of formula (I) according to Claim 1 for dyeing and/or printing wool, silk and synthetic polyamides.

8. Use of compounds of formula (I) according to Claim 1 for preparing printing inks for the InkJet process.
